# EUROPEAN PATENT APPLICATION

(11) **EP 3 871 510 A1**
(43) Date of publication of application: **01.09.2021**
(21) Application number: 20159391.0
(22) Date of filing: 25.02.2020
(51) Int. Cl.: A23D 7/00, A23D 7/005, A23D 7/01, A61K 31/352

(54) **MEDIUM CHAIN TRIGLYCERIDE BASED OIL PHASE IN WATER MICROEMULSIONS**

(71) Applicant: Tapperwein Collection AG, 9491 Ruggell (LI)
(72) Inventor: Pancurak, Frantisek, 08001 Presov (SK)
(74) Representative: Kaminski Harmann

(57) **Abstract**

The invention relates to an oil in water microemulsion comprising a water solubility agent composition designed for preparing a MCT based oil phase in water microemulsion. The water solubility agent composition is based on using sucrose ester as emulsifier and lecithin as co-emulsifier. The invention further relates to a method of emulsifying a MCT based oil phase to form an oil in water microemulsion, a food product comprising the MCT based oil phase in water microemulsion in diluted form and the use of a water solubility agent composition for dispersing an MCT based oil phase in water.

## Description

### -FIELD OF THE INVENTION-

The invention relates to a medium chain triglyceride (MCT) based oil phase in water microemulsion, a method of preparing a MCT based oil phase in water microemulsion, a food product comprising the MCT based oil phase in water microemulsion in diluted form and the use of a water solubility agent composition for dispersing MCT based oil phase in water.

### -BACKGROUND TO THE INVENTION-

Still nowadays, natural plants comprising biologically active constituents form the basis of a wide variety of medical or therapeutical treatments. The extraction and administration of these active constituents represents a key branch within the field of herbal medicine. A typical technique applied to extract/isolate desired active constituents of a natural plant is based on immersing the natural plant in a carrier oil. Thereby, the oil soluble active constituents are released, during the time of extraction, into the carrier oil either at room temperature or at elevated temperatures, e.g. around 70 °C. Typically, process parameters of the oil based extraction process are specifically chosen to selectively extract one desired active constituent or one desired group of active constituents. The as-extracted active constituents are bound in the carrier oil. The carrier oil comprises besides the extracted desired active constituents a wide variety of typically undesired natural side-constituents. Then, the carrier oil phase is further treated to purify and concentrate the desired active constituents. The hydrophobic carrier oil comprising the purified or concentrated active constituents can either directly be used or be combined into an ointment or balm formulation for external application.

Another approach, instead of isolating desired active constituents of natural plants, purify and concentrate them in a carrier oil phase, relates to extracting the full spectrum of the natural plant's constituents which are releasable into a carrier oil phase. This approach is based on the theory that a biological activity attributed to a plant's constituents is the result of a combinatorial effect generated by combining the full spectrum of the plant's constituents. A carrier oil phase comprising the full spectrum of a natural plant's constituents is typically referred to as full spectrum oil. For providing a full spectrum oil the parameters of the oil based extraction process are chosen in order to extract a majority of the plant's oil soluble constituents. This full spectrum oil then can either be directly applied without any further step of purification or concentration, or, depending on the final application, specific constituents can be removed or concentrated. The full spectrum oil comprises a full spectrum of oil soluble constituents which is plant specific and thereby can vary from plant to plant, e.g. due to climate exposure and growth conditions.

Besides these well-established forms of administration, there is a great interest in dispersing, by emulsification, the hydrophobic carrier oil phase in aqueous media. Thereby, other forms of administration are enabled, which are usually limited to water soluble active constituents. The targeted stability of the emulsion, concentration of the carrier oil phase in the emulsion, average size of the dispersed carrier oil phase in the continuous phase/liquid solvent and the need to conform with food administration regulations can make the emulsification/dispersion of a carrier oil phase challenging. Typical emulsion formulations comprise the carrier oil phase binding the active constituents, emulsifiers/surfactants, stabilizers and the solvent/continuous phase in which the carrier oil phase is dispersed/emulsified. Successful generation of an emulsion having the desired properties crucially depends on the selection and concentration of the compounds making up the emulsion formulation. Furthermore, an emulsion formulation typically is tailored to emulsify one specific carrier oil or carrier oil type from which the composition is well known and controllable. Such an emulsion formulation being designed to emulsify a specific carrier oil or carrier oil type is not expected to emulsify a carrier oil or carrier oil type being different from the one for which it is designed. For example, using a specifically tailored emulsion formulation, already slight variations in the composition of the carrier oil phase to be emulsified can result in a less stable/short-term stable or completely unstable emulsion. The development of emulsion formulations for preparing an oil in water emulsion can be a challenging task. For a given oil phase, e.g. carrier oil based phase, or type of oil phase the compounds making up the formulation have to be chosen according to specific requirements of the final application/product and the concentration of compounds has to be selected to enable a stable emulsion. Furthermore, the ratio of the amount of compounds needed for emulsifying a specific amount of oil phase to the specific amount of oil phase typically needs to be economically reasonable.

One specific class of carrier oils refers to the medium chain triglyceride (MCT) oils. MCT oils are commonly used as carrier oils in the production of natural/plant-derived full spectrum oils, as they are obtainable as odourless and food grade oils. Due to these advantageous properties of MCT oils, there is a need for dispersing such MCT based oil phases, for example comprising plant-derived full spectrum oils, in aqueous media to form a stable emulsion having properties specifically tailorable to a final application. Nevertheless, due to the nature of MCT based oil phases and e.g. plant-derived full spectrum oils their emulsification is particularly challenging.

What is missing are efficient emulsification routines providing for an efficient emulsification of such MCT based oil phases resulting in a long-term stable emulsion, wherein the emulsion forms the basis for administration of for example a plant-derived full spectrum oil.

It is therefore object of the invention to provide an emulsion formulation enabling an efficient emulsification of such MCT based oil phases in water resulting in a long-term stable emulsion.

### -SUMMARY OF THE INVENTION-

Herein disclosed is a MCT based oil phase in water microemulsion, a method of preparing an MCT based oil phase in water microemulsion, a food product comprising the MCT based oil phase in water microemulsion in diluted form and the use of a water solubility agent composition for preparing a MCT based oil phase in water microemulsion. Thereby the emulsification of purified or non-purified, concentrated or not concentrated plant-derived full spectrum oils is enabled. Furthermore, the microemulsion, the water solubility agent composition and the food product comply with standard food administration regulations.

The invention relates to an oil in water microemulsion comprising:
a. a medium chain triglyceride (MCT) based oil phase and
b. a water solubility agent composition,
wherein the water solubility agent composition comprises, water as main solvent, a sucrose ester as main emulsifier, a lecithin as co-emulsifier in combination with the sucrose ester, a polyol as co-solvent in combination with water, a monosaccharide as emulsification energy barrier reduction agent, and a short to medium chain alcohol as stabilizer, wherein the water solubility agent composition combines with the MCT based oil phase such that an oil in water microemulsion with the MCT based oil phase dispersed in water providing a d90 droplet size within the range of 10 to 100 nm is provided. Water can be tap water or deionized water.

The water solubility agent composition is specifically tailored to emulsify MCT based oil phases, for example comprising a plant-derived full spectrum oil. Thereby, a majority of a MCT based oil phase is formed by medium chain triglycerides. Medium chain triglycerides comprise medium chain fatty acids having 6 to 12 carbon atoms, e.g. such as caproic acid, caprylic acid, capric acid, lauric acid. Furthermore, MCT based oil phase can comprise MCT derived from sunflower or from fruits such as e.g. coconut, olive, avocado, grape etc. In order to homogeneously and stably disperse the MCT based oil phase in aqueous media it must be emulsified. In the emulsion the MCT based oil phase forms small droplets with an oil/water interface. Depending on the targeted form of administration the droplet size is essential. For example, for preparing soft drinks or other water based beverages the droplet size needs to be within a range which does not scatter visible light and therefore renders the soft drink or beverage clear/non-turbid and transparent. Typically, the droplet size distribution is influenced by the kind of emulsifier and its concentration with respect to the oil phase to be emulsified. In connection with MCT based oil phases sucrose esters are used, representing non-ionic emulsifiers, as main emulsifier to stabilize the thermodynamically unstable oil/water interface of the droplets rendering a key factor in enabling the formation of a long-term stable microemulsion. Sucrose esters specifically arrange at the oil/water interface and thereby induce a stabilization of the interface. Sucrose ester can be e.g. sucrose palmitate, sucrose mono-palmitate, sucrose laurate, sucrose mono-laurate, sucrose oleate, sucrose mono-oleate, sucrose myristate, sucrose mono-myristate, sucrose stearate and sucrose mono-stearate etc. In the microemulsion, the MCT based oil phase is homogeneously dispersed forming thermodynamically stable droplets having a d90 droplet size within the range of 10 to 100 nm, meaning that 90 percent by weight of the droplets have a diameter within the range of 10 to 100 nm.

A further component of the composition is lecithin. Lecithin can be derived from e.g. soy or sunflowers and synergistically combines with the sucrose esters to increase the stabilization of the oil/water interface.

A further factor in the formation of a microemulsion is the usage of a polyol, e.g. propylene glycol, glycerol etc., as co-solvent. The co-solvent acts together with the main solvent water, thereby forming the carrier/solvent liquid/continuous phase in which the MCT based oil phase is dispersed.

A further component of the composition is monosaccharide. A monosaccharide can e.g. be fructose, glucose or a mixture of fructose and glucose in the form of invert syrup. Its role in the formation of a long-term stable microemulsion relates to reducing the energy barrier to be overcome for the formation of an emulsion. This energy barrier is largely related to the amount of newly formed oil/water interface during emulsifying the MCT based oil phase. Thereby, the amount of newly formed oil/water interface is depending on the amount and size of the droplets. Therefore, typical recipes for preparing classic emulsions use water solubility agent compositions, which necessitate the introduction of energy, needed to overcome the considerable energy barrier, by high-energy mixing the emulsion with high speed mixers operated at speeds above 5000 rpm. In contrast, in the formation of microemulsions the use of monosaccharides and cosolvents in the disclosed water solubility agent composition renders a factor in reducing this energy barrier to a level, where low-energy or low-speed mixing or simple stirring, without applying high shear forces, at speeds below 1000 rpm is sufficient for emulsifying MCT based oil phases to form thermodynamically stable microemulsions.

A further component to the composition is short to medium chain alcohol. Short to medium chain alcohol can be e.g. sorbitol, maltitol or mono-n-alcohols like ethanol or n-butanol/butyl alcohol. Short to medium chain alcohol acts as stabilizer. Thereby stabilization relates mainly to influencing the structure/texture/viscosity of the water/solvent liquid/continuous phase and thereby its temperature induced solidification characteristics. This is important, for example, as a change in the state of aggregation of the water/solvent liquid/continuous phase could destabilize the emulsion.

The as-providable microemulsion can be generated using low-energy input, for example by simple stirring, is thermodynamically stable and can be used to provide, by dilution e.g. in water or water based media, clear transparent and long-term stable emulsions. Thereby, the production of food products e.g. beverages, such as fortified water or soft drinks or aqueous based sauces and dips is enabled. Furthermore, the composition provides for emulsifying MCT based oil phases with sucrose esters as main emulsifiers being food-grade and legally approved at specific levels in the related food applications, having low sensory profile, and tasteless properties. In addition, the composition provides for emulsifying MCT based oil phases with a weight ratio of sucrose esters to emulsifiable oil phase (SOR ratio) of less or equal to 1, making the emulsion generation cost efficient.

According to an embodiment the MCT based oil phase comprises a plant-derived full spectrum cannabidiol (CBD) oil.

Thereby, the plant-derived full spectrum CBD oil is derived from a natural plant, e.g. hemp plant, by full spectrum oil extraction techniques. The full spectrum oil extraction technique can relate to one of e.g. CO₂ extraction, distillation, liquid/liquid extraction, and liquid solid extraction. For the extraction typically a carrier oil is used. The plant-derived full spectrum CBD oil can be combined with other oil phases, e.g. aroma oils or further carrier oils such as e.g. seed oils, into the MCT based oil phase.

The MCT based oil phase can further comprise antioxidants, e.g. tocopherol acetate, which hinder oxidation of compounds bound in the MCT based oil phase and thereby conserve the compounds on a long term.

According to a further embodiment the sucrose ester is at least one of sucrose oleate, sucrose laurate, sucrose palmitate, sucrose myristate sucrose stearate, sucrose mono-oleate, sucrose mono-laurate, sucrose mono-palmitate, sucrose mono-myristate and sucrose mono-stearate, and the short to medium chain alcohol is sorbitol or n-butanol.

According to a further embodiment the sucrose ester is sucrose mono-palmitate or sucrose laurate, the polyol is propylene glycol or glycerol, and the monosaccharide is fructose or glucose.

According to a further embodiment the water solubility agent composition comprises a further polyol, and a further monosaccharide, wherein the polyol is propylene glycol, the further polyol is glycerol, the monosaccharide is fructose and the further monosaccharide is fructose or glucose.

According to a further advantageous embodiment the water solubility agent composition comprises a further sucrose ester, wherein the short to medium chain alcohol is n-butanol, the sucrose ester is sucrose mono-palmitate, and the further sucrose ester is sucrose mono-laurate. Then, sucrose mono-palmitate and sucrose mono-laurate are comprised in a sucrose mono-palmitate to sucrose mono-laurate weight ratio within the range of 1 to 2. The combination of these two sucrose esters has been proven to be specifically advantageous in the formation of long-term stable MCT based oil phase in water microemulsions, in particular wherein the MCT based oil phase comprises a plant-derived full spectrum CBD oil.

According to an embodiment the the plant-derived full spectrum CBD oil comprises a vegetable carrier oil and bound therein CBD and a full spectrum of oil bindable hemp plant extracts, in particular wherein the plant-derived full spectrum CBD oil is obtained by applying a full spectrum extraction technique to a hemp plant, the technique applied being one of CO2 extraction, distillation, liquid/liquid extraction, and liquid solid extraction.

According to an embodiment the vegetable carrier oil is an MCT based oil, derived from sunflower and/or at least one of the fruits coconut, olive, avocado and grape.

According to an advantageous embodiment the plant-derived full spectrum CBD oil comprises less than 1% tetrahydrocannabiol (THC) by weight of the total weight of the CDB oil and cannabinoids in a total concentration from about 10% to 70% by weight of the total weight of the CDB oil.

According to a specific embodiment the oil in water microemulsion comprises a water solubility agent composition comprising by weight of the total weight of the microemulsion: 10% to 70% water, 1% to 30% sucrose mono-palmitate, 1% to 60% propylene glycol, up to 20% glycerol, 1% to 60% fructose or a combination of fructose and glucose, up to 10% lecithin, up to 2% of a short to medium chain alcohol. Then the microemulsion comprises 1% to 20% by weight of the total weight of the microemulsion of a plant-derived full spectrum CBD oil comprised by the MCT based oil phase.

According to a further advantageous embodiment the oil in water microemulsion comprises sucrose ester and MCT based oil phase in a sucrose ester to MCT based oil phase weight ratio (SOR ratio) equal to or smaller than 1.

According to a further specific embodiment the oil in water microemulsion comprises a water solubility agent composition comprising by weight of the total weight of the microemulsion: 24% to 30% water, 8% to 14% sucrose mono-palmitate, 20% to 26% propylene glycol, 0.4% to 1% glycerol, 0.4% to 1% fructose, 3% to 9% lecithin derived from sunflower, up to 2% butyl alcohol. Then the microemulsion comprises 7% to 13% of a plant-derived full spectrum CBD oil comprised by the MCT based oil phase, wherein the full spectrum CBD oil comprises cannabinoids in a total concentration of 20% to 30% by weight of the total weight of the full spectrum CBD oil.

The invention further relates to a method of preparing a herein disclosed oil in water microemulsion comprising the steps of:
a. providing an MCT based oil phase as comprised by a disclosed microemulsion,
b. providing a first phase by combining water, polyol, monosaccharide, short to medium chain alcohol and sucrose ester into a containment under stirring at a temperature equal to or below 35 °C, in particular below 25°C,
c. providing a second phase by combining the MCT based oil phase and lecithin, and
d. mixing the second phase into the first phase aiding stirring to form a clear microemulsion.

In contrast to typically applied methods for emulsifying carrier oil based compositions the herein disclosed compositions provide for an emulsification routine which does not require any high-energy input homogenization step (or high speed mixing at rpm values above 5000) after mixing nor does the mixing have to be performed at elevated temperatures. Instead emulsification can be performed by simple stirring at temperatures around room temperature, e.g. within the range of 15°C to 35°C. Thereby, stirring relates to mixing at low rpm values in particular below 1000 rpm.

The invention further relates to a food product comprising a herein disclosed oil in water microemulsion in diluted form, in particular wherein the diluted form relates to a total concentration of sucrose ester in the food product of up to about 0.001 %, up to about 0.01 %, up to about 0.1 %, up to about 1 %, by weight of the total weight of the food product, and/or CBD in the food product of up to about 0.001 %, up to about 0.01 %, up to about 0.1 %, up to about 1 % by weight of the total weight of the food product.

The invention further relates to the use of a disclosed water solubility agent composition as comprised by a dislcosed microemulsion, for dispersing a disclosed MCT based oil phase, as comprised by a disclosed microemulsion, in water.

### -BRIEF DESCRIPTION OF THE DRAWINGS-

The preparation of a long-term stable MCT based oil phase in water microemulsion according to an embodiment of the invention is described below in more detail purely by way of example. In detail:

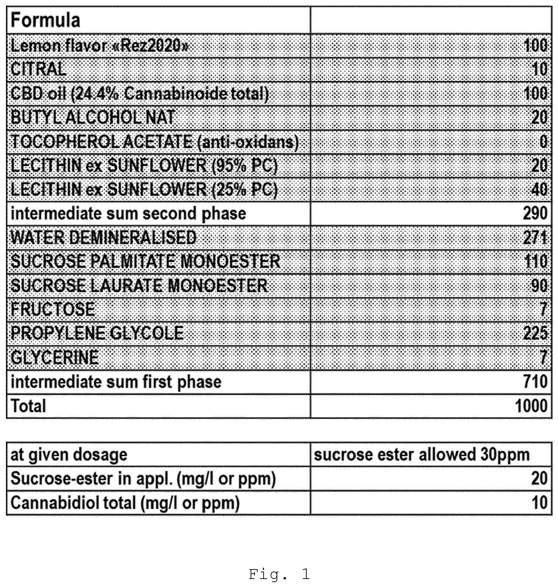

- Figure 1: shows an exemplary composition to be used for the preparation of a MCT based oil phase in water microemulsion and a food product;

### -DETAILED DESCRIPTION-

Figure 1 shows as an example a formulation forming a microemulsion in accordance with an embodiment of the invention. Thereby "Lemon flavor Rez2020" and "CITRAL" are aroma oils, CBD oil is a plant-derived full spectrum CBD oil comprising cannabinoids, and "Butyl alcohol nat" is n-butanol/butyl acohol, a natural short chain alcohol. Typically, the microemulsion is prepared by simple stirring.

Preparation of a first phase is performed by combining water soluble agents into a stainless steel vessel or similar containment at room temperature (max 35°C), equipped with a simple stirrer and comprises the steps of:
o Blending the solvents like water, propylene glycol, glycerol and if required, butyl alcohol;
o Dissolving the sucrose esters at preferably 30-35°C; and
o Dissolving monosaccharide.

Preparation of a second phase is performed by combining oil soluble agents and comprises the step of:
o Blending to the MCT based oil phase lecithin, the plant-derived full spectrum CBD oil and optionally aroma concentrate like citral.

Preparation of the microemulsion is performed by adding The second phase to the first phase little by little whilst stirring. After a certain time, a clear microemulsion is forming. The stirring time is depending on various factors, like batch size, container shape, stirrer efficiency and diameter. Such factors have to be determined specifically, according to the technical setup.

After the clear microemulsion has formed, the stirring has to be continued for at least 15, in particular 20 more minutes, in order to obtain a statistically good energy dissipation, and ensure the entire volume is well treated.The as prepared microemulsion comprising the plant-derived full spectrum CBD oil can then be used to produce a food product. Therefore, the as-prepared microemulsion can serve as stock solution and is diluteable to a desired degree. For example, by diluting a certain amount of the microemulsion in a certain volume of drinking water a water -fortified with the desired plant derived substances at the desired concentration range-is obtainable. Furthermore, by diluting the stock solution a beverage concentrate, e.g. a dosage syrup is obtainable. Thereby, a beverage can comprise the MCT based oil phase up to a concentration of 1 g/L or a beverage concentrate can comprise the MCT based oil phase in a concentration up to 100 g/L.

Preparing a beverage comprising at least part of the microemulsion, acidic ingredients should preferably be added last, after the desired amount of microemulsion has been well dispersed in the beverage. This is due to the fact, that pH values below 2.5 my affect the emulsion causing sediments or destabilization of the emulsion. Same reasoning applies for alcoholic ingredients.

## Claims

1. An oil in water microemulsion comprising:
a. a medium chain triglyceride (MCT) based oil phase and
b. a water solubility agent composition,
**characterized in that**
the water solubility agent composition comprises
c. water as main solvent,
d. a sucrose ester as main emulsifier,
e. a lecithin as co-emulsifier in combination with the sucrose ester,
f. a polyol as co-solvent in combination with water,
g. a monosaccharide as emulsification energy barrier reduction agent, and
h. a short to medium chain alcohol as stabilizer
wherein
the water solubility agent composition combines with the MCT based oil phase such that an oil in water microemulsion with the MCT based oil phase dispersed in water providing a d90 droplet size within the range of 10 to 100 nm is provided.

2. An oil in water microemulsion according to claim 1, wherein the MCT based oil phase comprises a plant-derived full spectrum cannabidiol (CBD) oil.

3. An oil in water microemulsion according to any of claims 1 to 2, wherein
a. the sucrose ester is at least one of sucrose oleate, sucrose laurate, sucrose palmitate, sucrose myristate sucrose stearate, sucrose mono-oleate, sucrose mono-laurate, sucrose mono-palmitate, sucrose mono-myristate and sucrose mono-stearate, and
b. the short to medium chain alcohol is sorbitol or n-butanol.

4. An oil in water microemulsion according to any of claim 1 to 3, wherein
a. the sucrose ester is sucrose mono-palmitate or sucrose laurate,
b. the polyol is propylene glycol or glycerol, and
c. the monosaccharide is fructose or glucose.

5. An oil in water microemulsion according to any of claims 1 to 4, wherein the water solubility agent composition comprises:
a. a further polyol, and
b. a further monosaccharide, and
wherein
the polyol is propylene glycol, the further polyol is glycerol, the monosaccharide is fructose and the further monosaccharide is fructose or glucose.

6. An oil in water microemulsion according to any of claims 1 to 5, the water solubility agent composition comprising:
a. a further sucrose ester,
wherein
b. the short to medium chain alcohol is n-butanol,
c. the sucrose ester is sucrose mono-palmitate, and the further sucrose ester is sucrose mono-laurate, and
d. sucrose mono-palmitate and sucrose mono-laurate are comprised in a sucrose mono-palmitate to sucrose mono-laurate weight ratio within the range of 1 to 2.

7. An oil in water microemulsion according to any of claims 2 to 6, wherein the plant-derived full spectrum CBD oil comprises a vegetable carrier oil and bound therein CBD and a full spectrum of oil bindable hemp plant extracts, in particular wherein the plant-derived full spectrum CBD oil is obtained by applying a full spectrum extraction technique to a hemp plant, the technique applied being one of CO₂ extraction, distillation, liquid/liquid extraction, and liquid solid extraction.

8. An oil in water microemulsion according to claim 7, wherein the vegetable carrier oil is an MCT based oil, derived from sunflower and/or at least one of the fruits coconut, olive, avocado and grape.

9. An oil in water microemulsion according to any of claims 2 to 8, wherein the plant-derived full spectrum CBD oil comprises less than 1% tetrahydrocannabiol (THC) by weight of the total weight of the full spectrum CDB oil and cannabinoids in a total concentration from about 10% to 70% by weight of the total weight of the full spectrum CDB oil.

10. An oil in water microemulsion according to any of claims 1 to 9, wherein the microemulsion comprises:
a. the water solubility agent composition comprising by weight of the total weight of the microemulsion:
(a) 10% to 70% water,
(b) 1% to 30% sucrose mono-palmitate,
(c) 1% to 60% propylene glycol,
(d) up to 20% glycerol,
(e) 1% to 60% fructose or a combination of fructose and glucose,
(f) up to 10% lecithin,
(g) up to 2% of a short to medium chain alcohol, and
b. 1% to 20% by weight of the total weight of the microemulsion of a plant-derived full spectrum CBD oil comprised by the MCT based oil phase.

11. An oil in water microemulsion according to any of claims 1 to 10, wherein the microemulsion comprises sucrose ester and MCT based oil phase in a sucrose ester to MCT based oil phase weight ratio equal to or smaller than 1.

12. An oil in water microemulsion according to any of claims 1 to 11, wherein the microemulsion comprises:
a. the water solubility agent composition comprising by weight of the total weight of the microemulsion:
(a) 24% to 30% water,
(b) 8% to 14% sucrose mono-palmitate,
(c) 20% to 26% propylene glycol,
(d) 0.4% to 1% glycerol,
(e) 0.4% to 1% fructose,
(f) 3% to 9% lecithin derived from sunflower,
(g) up to 2% butyl alcohol, and
b. 7% to 13% by weight of the total weight of the microemulsion of a plant-derived full spectrum CBD oil comprised by the MCT based oil phase, wherein the full spectrum CBD oil comprises cannabinoids in a total concentration of 20% to 30% by weight of the total weight of the full spectrum CBD oil.

13. A method of preparing an oil in water microemulsion according to any of claims 1 to 12, comprising the steps of:
a. providing an MCT based oil phase as comprised by the microemulsion according to any of claims 1 to 12,
b. providing a first phase by combining water, polyol, monosaccharide, short to medium chain alcohol and sucrose ester into a containment under stirring at a temperature equal to or below 35 °C, in particular below 25°C,
c. providing a second phase by combining the MCT based oil phase and lecithin, and
d. mixing the second phase into the first phase aiding stirring to form a clear microemulsion according to any of claims 1 to 12.

14. A food product comprising an oil in water microemulsion according to any of claims 7 to 13 in diluted form, in particular wherein the diluted form relates to a total concentration of
a. sucrose esters in the food product of up to about 0.001 %, up to about 0.01 %, up to about 0.1 %, up to about 1 % by weight of the total weight of the food product, and/or
b. CBD in the food product up to about 0.001 %, up to about 0.01 %, up to about 0.1 %, up to about 1 % by weight of the total weight of the food product.

15. Use of a water solubility agent composition as comprised by the microemulsion according to any of claims 1 to 12, for dispersing an MCT based oil phase, as comprised by the microemulsion according to any of claims 1 to 12, in water.
